# EUROPEAN PATENT SPECIFICATION

(11) **EP 1 296 639 B1**
(45) Date of publication and mention of the grant of the patent: **07.12.2005**
(21) Application number: 01923244.6
(22) Date of filing: 10.04.2001
(51) Int. Cl.: A61K 7/48, A61K 33/04, A61K 33/06, A61K 33/22, A61K 33/30

(54) **USE OF GREEN-LIGHT EMITTING MATERIALS IN PHARMACEUTICAL COMPOSITIONS**
VERWENDUNG VON GRÜN-LICHT EMITTIERENDEN MATERIALEN IN PHARMAZEUTISCHEN ZUSAMMENSETZUNGEN
L'UTILISATION DE MATERIAUX EMETTEURS DE LUMIERE VERTE DANS DES COMPOSITIONS PHARMACEUTIQUES

(30) Priority: 27.06.2000 US 604536
(43) Date of publication of application: 02.04.2003
(73) Proprietor: E-L Management Corp., New York, NY 10153 (US)
(72) Inventor: REIN, Glen, Huntington, NY 11743 (US); GEORGE, Liliana, Centerport, NY 11721 (US); CIOCA, Gheorge, Lake Grove, NY 11755 (US); COMOROSAN, Sorin, R-Bucharest 1 NR248 (RO)
(74) Representative: Hirsch & Associés
(86) International application number: PCT/US2001/011612
(87) International publication number: WO 2002/000190

(56) References cited:
- WO-A-00/56274
- WO-A-98/38981
- WO-A-99/29801
- FR-A- 1 528 072
- FR-A- 2 772 770
- GB-A- 2 209 624
- US-A- 5 562 763
- DATABASE CAPLUS [Online] CHEMICAL ABSTRACTS SERVICE, COLUMBUS, OHIO, US; retrieved from STN Database accession no. 1998:799831 XP002183856 & JP 10 330209 A (KOSEI CO) 15 December 1998 (1998-12-15)

## Description

### Field of the Invention

The invention relates to cosmetic and pharmaceutical compositions and methods of using same. More specifically, the invention relates to compositions having antioxidant, or free-radical scavenging, activity.

### Background of the Invention

Thousands of biochemical processes are ongoing in the living body at any given time. Many of these endogenous aerobic processes naturally give rise, as by-products, to very highly reactive molecules. A large number of these reactive molecules are known generally as free radicals, which are defined as an atom or group of atoms with an unpaired electron. However, other non-free radical reactive species are also generated by these processes. The processes that produce the reactive entities may be enzymatic, such as those involved in phagocytosis, respiration, the cytochrome P-450 system and prostaglandin synthesis; or they may be non-enzymatic, such as the reaction of oxygen with organic compounds, or reactions initiated by ionizing radiation. These reactive molecules, if uncontrolled, may rapidly, and randomly, react with molecules in their vicinity, giving rise to toxic products that can interfere with the body's normal physiological processes. However, the body has a number of defenses that can, under normal circumstances, to a large extent keep the potential damage resulting from these endogenous reactions in check.

There are a number of exogenous sources of these unwanted reactions as well. Many environmental agents, such as various dietary elements, pesticides, sunlight, tobacco smoke, air pollutants, anesthetics, and aromatic hydrocarbons, to which modern man is routinely exposed, also can generate highly reactive species. Thus, it is not surprising that the cumulative effects of these reactions can, and probably always eventually do, overwhelm the body's normal repair mechanisms. Considerable evidence exists that unchecked free radical reactions have some, if not major, involvement, in a number of disease states, for example, emphysema, inflammation, cancer, atherosclerosis and cataracts. Free radical reactions are also widely considered to have a major contributory effect on the natural aging process.

Among the most reactive of all regularly produced reactive species, and biologically among the most important, are those containing oxygen. These include, for example, partially reduced oxygen free radicals such as superoxide anion radicals, hydrogen peroxide and hydroxyl ions, as well as singlet oxygen. These reactive oxygen species have been implicated in a number of reactions that can cause serious damage to cellular components: for example, oxidizing radicals can attack the bases and sugar molecules of DNA, altering the molecular structure and thereby interfering with biological functions. They may also interact with unsaturated fatty acids in cell membranes, causing lipid peroxidation, which results not only in alteration of the protein:lipid interaction of the membrane, but in the production of breakdown products which can exert a host of undesired effects, such as inhibition of DNA synthesis, adenyl cyclase and glucose-6-phosphate, increase in capillary permeability and inhibition of platelet aggregation.

Because molecular oxygen is virtually everywhere and it freely accepts electrons, these oxygen-centered radicals are probably the most common mediators of cellular free radical reactions. They are of course routinely produced as a result of aerobic metabolism. However, a very significant amount is generated as a result of photochemical reactions. Many organic or inorganic compounds will absorb some UV radiation, and the absorbed energy will promote chemical reactions. There are a variety of recognized mechanisms by which light can cause the generation of oxygen-centered radicals; regardless of the mechanism, however, it is clear that the interaction of sunlight with organic or inorganic substrates on exposed skin can result in one or more reactive oxygen species being produced on the skin.

It has been recognized in recent years that the presence of oxygen radicals on the skin is probably responsible for a number of the undesirable effects of prolonged exposure to the sun. For example, the aging phenomenon generally observed throughout the body is frequently observed prematurely on the skin as a result of photoaging, which accelerates the process of deterioration of elastin and collagen, among other effects. There is also an increased risk of skin cancer of all types. In response to this need, the skin care industry has continued to seek new and more effective antioxidants, i.e., compounds which will in some way interfere with oxidative processes mediated by free radicals. Antioxidants can act by either preventing a reaction completely, or by breaking the already initiated chain of steps in the reaction. Many compounds, for example, phenols or sulfides, are known to be capable of interfering with oxidative processes; however, such compounds may not be appropriate for use on the skin, or may be difficult to formulate in a cosmetically or pharmaceutically acceptable manner.

The present invention now provides a new antioxidant composition based on a novel concept and observation relating to the use of visible light to inhibit or scavenge free radicals on the skin.

### Summary of the Invention

The invention relates to the use of a composition comprising at least one compound providing a source of green light for the production of pharmaceutical compositions for the treatment of oxygen free radical damage in the skin.

Particularly the composition comprises a green phosphorescent material, a green fluorescent material, or a combination thereof.

Another object of the invention is the use of a composition comprising at least one compound providing a source of green light for the production of pharmaceutical compositions for the treatment of the effects of UV exposure on the skin.

The green light apparently can quench or prevent the generation of free radicals, thereby protecting the skin to which it is applied from free-radical damage, and also protecting the composition from the potentially decaying effects of free radicals present in the composition. Particularly preferred for use in the composition is a combination of a green phosphorescent material with a green fluorescent material, which composition can be activated by simple exposure to daylight.

### Brief Description of the Figures

Figures 1-4 illustrate the effect of various sources of green light on the level of oxygen free radicals under various conditions: Figure 1: green phosphorescent pigment; Figure 2: green lamp with ambient lighting; Figure 3: green lamp with low ambient lighting; Figure 4, green lamp in a dark box.

### Detailed Description of the Invention.

The ability of green light to alter the chemical behavior of certain materials has previously been disclosed. Known as the "Comorosan effect", it has been shown that irradiation of a dry enzyme substrate with green light (λ=546 nm, or in that region), with specified exposure times, prior to its dissolution results in an increase in the reaction rates of the enzyme(Comorosan et al., Physiol. Chem. and Physics 3: 343, 1971; Comorosan, Int. J. Quantum Chem.: Quantum Biology Symp. I: 221, 1974; Comorosan et al., Physiol. Chem. and Physics 12: 497, 1980). A similar effect, but in reverse, was observed when a film developer was so irradiated, leading to a decrease in film opacity for the irradiated material(Peschel et al., Physiol. Chem. Physics and Med. NMR 19: 271-274, 1987). While interesting observations, however, they have not led to widespread practical commercial application of the phenomenon.

It has now been unexpectedly discovered that green light exposure has an antioxidant effect in chemical compositions. More specifically, in experiments expressly designed to generate peroxide radicals, it has been found that treatment of the free radical-generating composition with green light results in a reduction in the level of free radicals found in the treated composition relative to the control. The effects observed are particularly interesting for two reasons. First, unlike the Comorosan effect which occurs in the solid state, it is the first time that this effect has been transmitted to an aqueous system. Second, the source of the green light does not affect the efficacy of the treatment: the green light can be from an external source, i.e., a lamp which is used to irradiate the composition, or an internal source, i.e., a green-light emitting substance incorporated into the composition. In both cases, the free radicals are substantially reduced. An additional surprising aspect of the invention is that the observed treatment is not limited to specific exposure times, unlike previous observations of this phenomenon, wherein the activity of green light was only seen during critical exposure times. In contrast, the present effect of green light can be obtained from prolonged exposure of the green light to the treated substrate, with no diminution of activity, and is not critically dependent on specific exposure times.

In a preferred embodiment, the treatment of the composition is accomplished by including a green light source directly in the composition. This is most readily accomplished by the incorporation of a green phosphorescent pigment into the composition. By "phosphorescent pigment" in the present context is meant not only phosphorescent pigments in the traditional sense, such as are enumerated below, but also any cosmetically acceptable materials that emit phosphorescent light in the wavelength range of from about 500-550 nm. The presence of the phosphorescent pigment provides a continuous source of the green light in the composition; the phosphorescence is activated by exposure to the UV component of ordinary light, the effect of which lingers for several hours. There are several different compounds known to possess the property of green phosphorescence, including, but not limited to, zinc sulfide(doped with copper and/or manganese), strontium aluminate or phosphorescent calcite, ceramic oxides doped with rare earth elements such as europium, terbium, or dysprosium, zinc boro-silicates(glass) doped with copper or rare earth elements, metals such as molybdenum plated with copper or rare earth elements, and cesium salts. Particularly preferred for use in the invention are zinc sulfide and strontium aluminate.

In practical use, the pigments can be used as is, or they can be coated or microencapsulated in order to enhance their photostability or to facilitate their dispersion in the formulation. Coatings used for the pigments can be any that are typically used to coat traditional cosmetic pigments. Examples of useful pigment coatings are hydrophobic coatings such as methicone, dimethicone, silanes, polyethylene, metal soaps, lecithin, waxes, nylon or fluorochemicals. Examples of hydrophilic coatings include dimethicone copolyol. The nature of the coating will depend on the type of vehicle chosen. The amount of pigment employed depends on the phosphorescent strength of the chosen pigment, but normally will be about 0.01 to about 50%, preferably about 1 to about 10%, by weight of the total composition, with the amounts at the higher end of the range normally being particularly useful in the case in which the pigment is coated.

In a particularly preferred embodiment, the phosphorescent pigment is combined together with a source of fluorescent green light. A preferred source of green fluorescence are powders of fluorescent minerals. Especially preferred are minerals that produce a green to bluish green fluorescence; minerals of this type include, but are not limited to, andalusite and chiastolite(aluminum silicate); amblygonite(basic lithium aluminum phosphorate); phenakite(beryllium silicate); variscite(hydrous aluminum phosphate); serpentine(basic magnesium silicate); amazonite(potassium aluminum silicate); amethyst(silicon dioxide); chrysoberyl(beryllium aluminum oxide); turquoise(copper-containing basic aluminum phosphate); colorless, yellow or pink tourmaline(borosilicate); amber(succinite/various resins); opal(hydrous silicon dioxide); cerussite (lead carbonate); fuchsite(potassium aluminum silicate); diopside(calcium magnesium silicate); ulexite(hydrous sodium calcium borate); aragonite (calcium carbonate); and willemite(zinc silicate). Particularly preferred among these are the silicates, particularly those with a strong fluorescence, such as fuchsite, diopside, ulexite, aragonite and willemite.

The fluorescent powders can be prepared by standard grinding techniques, such as jet milling, roller milling or pulverization. The average particle size of the powders will normally be, for aesthetic reasons, no larger than about 45µ; preferably the particle size is between .5-20µ, and more preferably between about .5 and 5µ, with the harder minerals preferably being ground within the lower end of the recommended range. The amounts of the powders may be varied depending upon the intensity of the fluorescence and color of the mineral, and can be present in an amount of from about 0.01% to about 50%, more preferably, however, the amount used will be between about 0.01% up to about 10%.

Although fluorescent mineral powders are particularly preferred, other fluorescent materials can also be used. Examples include but are not limited to fluorescent biomolecules, such as green fluorescent protein, fluorescent plankton or fluorescent organic or inorganic dyes, which optionally may be bound to a polymer. These materials can be employed in amounts comparable to those for the fluorescent mineral powders, taking into account their relative fluorescent strength.

The green light source can be incorporated into any convenient form typically used for cosmetic formulations. Examples of useful form include hot pour formulations, water-in-oil emulsions, oil-in-water emulsions, gels, sticks, sprays, anhydrous formulations, and pressed or loose powders. The product can also be a makeup product or a skin care product. The incorporation of the green-light producing component can achieve a number of different effects in a topical formulation. As first noted, it can of course be used as an antioxidant, whereby it achieves two possible effects. First, its presence will retard or prevent the degradation of the formula in which it is incorporated, i.e., it acts as a preservative. It also acts to protect the skin to which the formulation is applied, by reducing or scavenging the number of oxygen free radicals that would ordinarily occur on the skin by normal or prolonged exposure to environmental stimuli of free radical generation. Similarly, it has a protecting effect on skin cells against the damaging effects of UV radiation.

The compositions can be applied in a number of ways, depending on the intended use of the final product. For example, the compositions can be used as a routine antioxidant treatment for the skin, whereby the application prevents or alleviates any free-radical damage that might occur due to everyday exposure to free-radical generating stimuli. A preferred method of obtaining the benefits of the composition for this purpose is therefore a chronic application. For example, topical application of the composition, in an amount of from about 0.1 mg/cm² to 2 mg/cm² of skin, will be performed from about once per week to about 4 or 5 times daily, preferably from about 3 times a week to about 3 times daily, most preferably about once or twice per day. By "chronic" application, it is meant herein that the period of topical application may be over the lifetime of the user, preferably for a period of at least about one month, more preferably from about three months to about twenty years, more preferably from about six months to about ten years, more preferably still from about one year to about five years, thereby in the short-term, resulting in the immediate reduction of free-radical presence on the skin, and ultimately, in the long term, resulting in the treatment or prevention of both the internal and external signs of photo- or chronoaging.

The compositions, as noted, can also be used to treat or prevent the damaging effects of UV radiation. In this embodiment, the composition can be applied as described above, as a regular protection against casual UV exposure. In addition, however, in this use, it will be preferred to apply the composition before exposure to sunlight, for example, when the user anticipates a long period of outdoor activity with considerable time spent in the sun, and/or during the time of such exposure.

The invention will be further illustrated by the following nonlimiting examples.

### EXAMPLES

### Example 1

This example illustrates the effect of green light in reducing the amount of oxygen free-radicals in a free-radical generating environment.

To test the antioxidant properties of green light, two sources of green light are evaluated. Green light is generated using a strontium aluminate phosphorescent pigment, as well as a mercury vapor lamp filtered with a 547 nanometer filter. For the studies involving the pigment, a reaction cuvette is surrounded on all sides by four cover slips and secured within a jar which is covered with a thin sheet of aluminum foil. Each cover slip is coated with the pigment, while uncoated coverslips are used as the control. Pigment-coated coverslips are activated for 10 minutes using a UVA lamp immediately before assembly in the jar.

Experiments involving the lamp-generated green light use two experimental setups. The first "dark" setup is done inside a cardboard box where the cuvette is irradiated from above. The second "ambient" setup is done either in full ambient light or low ambient light(cuvette loosely covered with foil) while being irradiated from below. The lamp is on during control treatments but the light is blocked and does not reach the cuvette. In both cases, temperature differences between control and treatment conditions are less than or equal to 0.4°C.

To demonstrate antioxidant activity, superoxides are chemically generated from hydrogen peroxide and the effect of green light on the concentration of superoxides is measured. The amount of superoxides generated from hydrogen peroxide can be measured by their binding to Lucigenin, in an assay conducted as follows. NaOH(0.25 ml, 4N) is incubated with H₂O₂ (1ml, 0.6%) in the presence of Tween (1ml, 2%) in 4% ethanol. The reaction is carried out in a closed quartz cuvette with a membrane containing screw cap. After 6 minutes of gentle shaking (using a Titertek shaker from Flow Labs at setting #6), Lucigenin (0.5ml, 10 mM) is injected through the membrane using a 25 gauge syringe. The fluorescence spectra is obtained by exciting the solution at 465 nm and monitoring the emission(using the kinetic program) every minute for six minutes at 508 nm. The slope is calculated and used as the final numerical data value. The slope is calculated and used as the final numerical data value for statistical analysis. Suboptimal concentrations (0.6%) of H₂O₂ are used to increase the sensitivity of the assay.

In testing the activity of green light, superoxide free radicals are generated as described above for six minutes. During the six minutes, the reaction cuvette is exposed to green light or not (control). After the test period, the free radicals are quantified by adding Lucigenin and measuring the kinetics (for six minutes) of fluorescent light emission at 508nm. For the lamp experiments, a given experimental run consists of one control and one treatment. The order of the pair is changed randomly. For the pigment experiments a series of controls are all run, and then a series of treatments. Quantitative information is obtained from the kinetics of Lucigenin fluorescence after the free radicals are generated(Figures 1-4).

For experiments with the lamp, a separate group of experiments is conducted, in which delta values are calculated. Delta values are obtained as the absolute value of the numerical subtraction of control and treatment fluorescence for each pair in a given run. For the ambient environment, fluorescent values at six minutes are used and for the dark environment fluorescence at three minutes. Control deltas are obtained from a separate set of experiments where each pair of runs consists of two unexposed controls. In all experiments, treatment deltas are always negative, indicating the green light exposure is always effective as an antioxidant, although the magnitude of the response varies from run to run. The results are all statistically significant, with p<0.0001 for the pigment, and p=0.013 for full ambient light plus green light, p=0.014 for low ambient light plus green light, and p=.009 for dark plus green light. Thus, the antioxidant effect is seen under all light conditions; however, green light in the presence of low ambient light appears most effective and most reproducible, the effect being somewhat less reproducible, but nonetheless statistically significant, in full ambient light and in darkness.

A follow-up experiment is conducted in which the delay between the addition of Lucigenin and the data collection is eliminated, and slope is calculated immediately after adding Lucigenin. Superoxide free radicals are generated from the chemical oxidation of hydrogen peroxide in the presence of NaOH and Tween/ethanol inside a cuvette for 12 minutes. Lucigenin is then added and fluorescence at 508nm is monitored for the next 3 minutes. A mercury vapor lamp is used as the source of the green light, which is shined on the cuvette for varying amounts of time during the 12 minutes of free radical generation. Control runs did not receive the green light, and were exposed to the same heat and light conditions as the experimental runs. Slopes are calculated and averaged over all individual experiments. As a positive control the test was also run with BHT, a known antioxidant. The results are shown in Table 1.

**Table 1**

| | Average Slope | Standard Deviation | n | p |
|---|---|---|---|---|
| Control | +101.4 | 18 | 7 | |
| 3 min. green light | -22.5 | 13 | 4 | <0.001 |
| 6 min. green light | -17.0 | 17 | 6 | <0.001 |
| 9 min. green light | -27.0 | 16 | 6 | <0.001 |
| 12 min. green light | -7.5 | 17 | 4 | <0.001 |
| 0.1% BHT | -27.3 | 1.8 | 2 | |
| 0.05% BHT | -15.0 | 2.8 | 2 | |
| 0.025% BHT | -1.9 | 0.7 | 2 | |

The results confirm the antioxidant activity shown in the first experiment, and show that this activity is comparable to that shown by BHT.

### Example 2.

This experiment shows the effect of green light produced by a phosphorescent pigment in protecting skin cells from UV damage.

Mouse melanocyte cultures (BC1) are treated with green light emitted from a strontium aluminate pigment. After prior activation of a pigment-coated surface with UVA light, petri dishes containing the cells are placed on top of the pigment. Five hours later the cells are exposed to UVB light and then immediately exposed to the freshly activated pigment. The cells are then exposed to four additional light treatments over the next 24 hours. At that point the cells are treated with neutral red and standard procedures followed to quantitate cell viability. Each experimental condition is done in quadruplicate with the final cell viability value calculated as the average(absorption at 540nm) of these four samples. Data are also expressed as a percent decrease in cell viability relative to controls that are untreated with green light or UV light. The experiment is repeated for confirmation of results. The results are shown in Table 2.

**Table 2**

| | Average Cell Viability | SD | % change | Statistical Significance w/r/t control |
|---|---|---|---|---|
| Control | 0.891 | 0.05 | | |
| UVB 10mJ | 0.626 | 0.06 | -29.7 | p<0.0001 |
| UVB + green light | 0.806 | 0.09 | -10.5 | NS |
| | | | | |
| Control | 0.445 | 0.02 | | |
| UVB 20mJ | 0.119 | 0.005 | -73.2 | p<0.0001 |
| UVB + green light | 0.228 | 0.01 | -48.8 | p<0.0001 |

The results indicate that the green light has a protective effect against UV damage. However, the magnitude of the protective effect is dependent on the dose, with green light being less protective against higher doses of UVB.

### Example 3

This example illustrates the effect of green light produced by a second phosphorescent pigment in increasing cell viability.

Mouse melanocyte cultures (BC1) are treated with green light emitted from a zinc sulfide pigment. After prior activation of a pigment-coated surface with UV-A light for 10 minutes, as described above, petri dishes containing the cells are placed on top of the activated pigment. For most experiments, melanocytes are pretreated for 1-2 days by re-activating the pigment every 3-4 hours. The cells are exposed to 10mJ/cm² or 20mJ/cm² UVB light and then exposed to four additional green light treatments over the next 24 hours. At that point the cells are treated with neutral red and the cell viability is evaluated using the neutral red dye uptake assay. Each experimental condition is done in quadruplicate with the final cell viability value calculated as the average absorption at 540 nm for these four samples. Data are expressed as a percent decrease in cell viability relative to untreated controls. Statistical significance is determined for cells treated with UV alone vs. UV plus green light. Results are shown in Table 3.

**Table 3**

| | Cell Viability | SD | % change | Statistical Significance w/r/t control |
|---|---|---|---|---|
| **10mJ/cm**^{**2**} | | | | |
| Control | 0.891 | 0.05 | | |
| UVB | 0.626 | 0.06 | -29.7 | |
| UVB+green light | 0.806 | 0.09 | -10.5 | p=0.023 |
| | | | | |
| Control | 0.647 | 0.02 | | |
| UVB | 0.482 | 0.009 | -25 | |
| UVB+green light | 0.682 | 0.02 | -9.3 | P<.001 |
| | | | | |
| Control | 0.752 | 0.04 | | |
| UVB | 0.608 | 0.02 | -19 | |
| UVB+green light | 0.682 | 0.02 | -9.3 | p=0.004 |
| | | | | |
| Control | 0.668 | 0.02 | | |
| UVB | 0.565 | 0.01 | -15 | |
| UVB+green light | 0.630 | 0.03 | -5.7 | p<0.001 |

| **20mJ/cm**^{**2**} | | | | |
|---|---|---|---|---|
| Control | 0.445 | 0.02 | | |
| UVB | 0.119 | 0.005 | -73 | |
| UVB+green light | 0.228 | 0.01 | -49 | p<0.001 |
| | | | | |
| Control | 0.755 | 0.01 | | |
| UVB | 0.373 | 0.01 | -50 | |
| UVB+green light | 0.483 | 0.04 | -36 | p<0.004 |

The results indicate that in all cases UV-induced damage, as measured by decreased cell viability, is significantly reduced by the presence of green light. Green light is more effective in providing this protection against low dosages of UVB, but significant effects are also observed at higher doses.

## Claims

1. The use of a composition comprising at least one compound providing a source of green light for the production of pharmaceutical compositions for the treatment of oxygen free radical damage in the skin.

2. The use of claim 1 in which the composition comprises a green phosphorescent material, a green fluorescent material, or a combination thereof.

3. The use of claim 2 in which the phosphorescent material is selected from the group consisting of zinc sulfide doped with copper or manganese or a combination thereof, strontium aluminate, phosphorescent calcite, ceramic oxides doped with a rare earth element, zinc boro-silicates doped with copper or a rare earth element, metals plated with copper or a rare earth element, and cesium salts.

4. The use of claim 2 or 3 in which the fluorescent material is a mineral powder.

5. The use of any one of claims 1 to 4 in which the composition comprises zinc sulfide or strontium aluminate, and a fluorescent mineral powder.

6. The use of any one of claims 1 to 5 in which the composition comprises a green phosphorescent material.

7. The use of a composition comprising at least one compound providing a source of green light for the production of pharmaceutical compositions for the treatment of the effects of UV exposure on the skin.

8. The use of claim 7 in which the composition is applied before UV exposure.

9. The use of claim 7 or 8 in which the composition comprises a phosphorescent material selected from the group consisting of zinc sulfide, doped with copper or manganese or a combination thereof, strontium aluminate, phosphorescent calcite, ceramic oxides doped with a rare earth element, zinc borosilicates doped with copper or a rare earth element, metals plated with copper or a rare earth element, and cesium salts.

10. The use of any one of claims 7 to 9 in which the composition comprises a fluorescent material selected from the group consisting of fluorescent mineral powders, fluorescent biomolecules, and fluorescent organic and inorganic dyes.

11. The use of any one of claims 7 to 10 in which the composition comprises from about 0.01 to about 50% of a phosphorescent material.

12. The use of any one of claims 7 to 11 in which the composition comprises from about 1 to about 10% of a phosphorescent material.

13. The use of any one of claims 7 to 12 in which the phosphorescent material is zinc sulfide or strontium aluminate.

14. The use of any one of claims 7 to 13 in which the composition comprises at least one fluorescent mineral powder.

15. The use of any one of claims 7 to 14 in which the composition comprises both a phosphorescent material and a fluorescent material.

## Patentansprüche

1. Verwendung einer Zusammensetzung aufweisend wenigstens eine eine Quelle grünen Lichts zur Verfügung stellende Verbindung zur Produktion pharmazeutischer Verbindungen zur Behandlung einer sauerstofffreien radikalischen Schädigung in der Haut.

2. Verwendung nach Anspruch 1, in welcher die Zusammensetzung ein grünes phosphoreszierendes Material, ein grünes fluoreszierendes Material oder eine Kombination davon aufweist.

3. Verwendung nach Anspruch 2, in welcher das phosphoreszierende Material aus einer Gruppe gewählt ist, bestehend aus: Zinksulfid dotiert mit Kupfer oder Mangan oder einer Kombination davon, Strontiumaluminat, phosphoreszierendem Kalzit, keramischen Oxiden dotiert mit einem Seltenerdelement, Zinkborosilikat dotiert mit Kupfer oder einem Seltenerdelement, Metallen beschichtet mit Kupfer oder einem Seltenerdelement und Caesiumsalzen.

4. Verwendung nach Anspruch 2 oder 3, in welcher das fluoreszierende Material ein Mineralpulver ist.

5. Verwendung nach einem der Ansprüche 1 bis 4, in welcher die Zusammensetzung Zinksulfid oder Strontiumaluminat und ein fluoreszierendes Mineralpulver aufweist.

6. Verwendung nach einem der Ansprüche 1 bis 5, in welcher die Zusammensetzung ein grünes phosphoreszierendes Material aufweist.

7. Verwendung einer Zusammensetzung aufweisend wenigstens eine eine Quelle grünen Lichts zur Verfügung stellende Verbindung zur Produktion pharmazeutischer Verbindungen zur Behandlung der Auswirkungen einer UV-Belichtung auf der Haut.

8. Verwendung nach Anspruch 7, in welcher die Zusammensetzung vor der UV-Belichtung angewendet wird.

9. Verwendung nach Anspruch 7 oder 8, in welcher die Zusammensetzung ein phosphoreszierendes Material aufweist ausgewählt aus der Gruppe bestehend aus Zinksulfid dotiert mit Kupfer oder Mangan oder einer Kombination davon, Strontiumaluminat, phosphoreszierendem Kalzit, keramischen Oxiden dotiert mit einem Seltenerdelement, Zinkborosilikaten dotiert mit Kupfer oder einem Seltenerdelement, Metallen beschichtet mit Kupfer oder einem Seltenerdelement und Caesiumsalzen.

10. Verwendung nach einem der Ansprüche 7 bis 9, in welcher die Zusammensetzung ein fluoreszierendes Material ausgewählt aus der Gruppe bestehend aus einem fluoreszierenden Mineralpulver, fluoreszierenden Biomolekülen und fluoreszierenden organischen und anorganischen Farbstoffen aufweist.

11. Verwendung nach einem der Ansprüche 7 bis 10, in welcher die Zusammensetzung zwischen etwa 0,01 bis 50 % eines phosphoreszierenden Materials aufweist.

12. Verwendung nach einem der Ansprüche 7 bis 11, in welcher die Zusammensetzung zwischen etwa 1 bis etwa 10 % eines phosphoreszierenden Materials aufweist.

13. Verwendung nach einem der Ansprüche 7 bis 12, in welcher das phosphoreszierende Material Zinksulfid oder Strontiumaluminat ist.

14. Verwendung nach einem der Ansprüche 7 bis 13, in welcher die Zusammensetzung wenigstens ein fluoreszierendes Mineralpulver aufweist.

15. Verwendung nach einem der Ansprüche 7 bis 14, in welcher die Zusammensetzung sowohl ein phosphoreszierendes Material als auch ein fluoreszierendes Material aufweist.

## Revendications

1. Utilisation d'une composition comprenant au moins un composé fournissant une source de lumière verte pour la production de compositions pharmaceutiques destinées au traitement de lésions provoquées par des radicaux libres d'oxygène dans la peau.

2. Utilisation selon la revendication 1, dans laquelle la composition comprend un matériau phosphorescent vert, un matériau fluorescent vert ou une combinaison de ceux-ci.

3. Utilisation selon la revendication 2, dans laquelle le matériau phosphorescent est choisi dans le groupe consistant en sulfure de zinc dopé avec du cuivre ou du manganèse ou une combinaison de ceux-ci, aluminate de strontium, calcite phosphorescente, oxydes céramiques dopés avec un élément des terres rares, borosilicates de zinc dopé avec du cuivre ou un élément des terres rares, métaux plaqués avec du cuivre ou un élément des terres rares, et sels de césium.

4. Utilisation selon la revendication 2 ou 3, dans laquelle le matériau fluorescent est une poudre minérale.

5. Utilisation selon l'une quelconque des revendications 1 à 4, dans laquelle la composition comprend du sulfure de zinc ou de l'aluminate de strontium, et une poudre minérale fluorescente.

6. Utilisation selon l'une quelconque des revendications 1 à 5, dans laquelle la composition comprend un matériau phosphorescent vert.

7. Utilisation d'une composition comprenant au moins un composé fournissant une source de lumière verte pour la production de compositions pharmaceutiques destinées au traitement des effets d'une exposition aux UV sur la peau.

8. Utilisation selon la revendication 7, dans laquelle la composition est appliquée avant une exposition aux UV.

9. Utilisation selon la revendication 7 ou 8, dans laquelle la composition comprend un matériau phosphorescent choisi dans le groupe consistant en sulfure de zinc dopé avec du cuivre ou du manganèse ou une combinaison de ceux-ci, aluminate de strontium, calcite phosphorescente, oxydes céramiques dopés avec un élément des terres rares, borosilicates de zinc dopé avec du cuivre ou un élément des terres rares, métaux plaqués avec du cuivre ou un élément des terres rares, et sels de césium.

10. Utilisation selon l'une quelconque des revendications 7 à 9, dans laquelle la composition comprend un matériau fluorescent choisi dans le groupe consistant en poudres minérales fluorescentes, biomolécules fluorescentes, et colorants organiques ou inorganiques fluorescents.

11. Utilisation selon l'une quelconque des revendications 7 à 10, dans laquelle la composition comprend d'environ 0,01 à environ 50% d'un matériau phosphorescent.

12. Utilisation selon l'une quelconque des revendications 7 à 11, dans laquelle la composition comprend d'environ 1 à environ 10% d'un matériau phosphorescent.

13. Utilisation selon l'une quelconque des revendications 7 à 12, dans laquelle le matériau phosphorescent est du sulfure de zinc ou de l'aluminate de strontium.

14. Utilisation selon l'une quelconque des revendications 7 à 13, dans laquelle la composition comprend au moins une poudre minérale fluorescente.

15. Utilisation selon l'une quelconque des revendications 7 à 14, dans laquelle la composition comprend à la fois un matériau phosphorescent et un matériau fluorescent.
